# EUROPEAN PATENT APPLICATION

(11) **EP 0 570 111 A1**
(43) Date of publication of application: **18.11.1993**
(21) Application number: 93302984.5
(22) Date of filing: 19.04.1993
(51) Int. Cl.: C09B 57/00, C07D 493/04

(54) **Polycyclic dyes**

(30) Priority: 11.05.1992 GB 9210116
(71) Applicant: ZENECA LIMITED, London W1Y 6LN (GB)
(72) Inventor: Hall, Nigel, Greenmount, Bury BL8 4DH (GB); Usher, Michelle Ann, Prestwich, Manchester M25 8HQ (GB)
(74) Representative: Giles, David Eric

(57) **Abstract**

A polycyclic dye of the Formula (1): 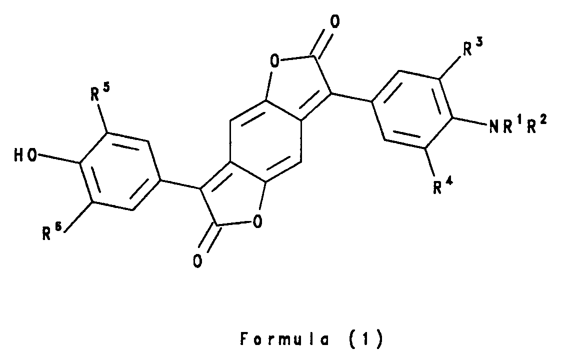
wherein:
- R¹ and R²: each independently is alkyl, alkenyl, cycloalkyl, aryl or aralkyl each of which may be optionally substituted or -H; or
- R¹ and R²: together with the N atom to which they are attached form a heterocyclic ring; or
- R¹ and R²: together with the N atom to which they are attached and the adjacent carbon atom of the benzene ring to which R³ or R⁴ is attached form a heterocyclic ring fused to the benzene ring;
- R³ and R⁴: each independently is -H or an optionally substituted alkyl group, an optionally substituted alkoxy group or halogen provided that if both R³ and R⁴ are alkyl or alkoxy that one of R¹ and R² is H; and
- R⁵ and R⁶: each independently is -H or optionally substituted alkyl.

The dyes are useful for the coloration of synthetic fibres, particularly polyester fibres, using disperse dyeing techniques and build up well giving blue shades which exhibit good levelness and excellent light fastness.

## Description

This specification describes an invention which relates to certain novel polycyclic dyes which have improved dyeing properties.

According to the present invention there is provided a polycyclic dye of the Formula (1): 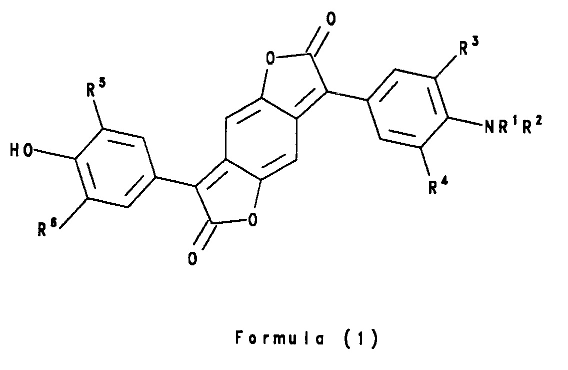
wherein:
- R¹ and R²: each independently is alkyl, alkenyl, cycloalkyl, aryl or aralkyl each of which may be optionally substituted or -H; or
- R¹ and R²: together with the N atom to which they are attached form a heterocyclic ring; or
- R¹ and R²: together with the N atom to which they are attached and the adjacent carbon atom of the benzene ring to which R³ or R⁴ is attached form a heterocyclic ring fused to the benzene ring;
- R³ and R⁴: each independently is -H or an optionally substituted alkyl group, an optionally substituted alkoxy group or halogen provided that if both R³ and R⁴ are alkyl or alkoxy that one of R¹ and R² is H; and
- R⁵ and R⁶: each independently is -H or optionally substituted alkyl.

A preferred sub-group of polycyclic dyes of Formula (1) is that in which R¹ and R² each independently is alkyl, alkenyl, cycloalkyl, aryl or aralkyl each of which may be optionally substituted or -H and in which R³, R⁴, R⁵ and R⁶ are as hereinbefore defined.

R¹ and R² each independently are preferably -H or alkyl. R³ and R⁴ each independently are preferably -H, alkyl or alkoxy. R⁵ and R⁶ are preferably -H.

When any one of R¹, R², R³, R⁴, R⁵ and R⁶ is alkyl it preferably contains up to 20, more preferably up to 10 carbon atoms and especially up to 4 carbon atoms. The alkyl groups may be straight or branched chain alkyl groups.

When R¹ or R² is alkenyl it preferably contains from 2 to 20, more preferably from 2 to 10 and especially 3 or 4 carbon atoms; when R¹ or R² is cycloalkyl it is preferably C₄₋₈-cycloalkyl, especially cyclohexyl; and when R¹ or R² is aryl or aralkyl, the aryl is preferably monohomocyclic aryl, i.e. phenyl and the aralkyl is preferably phenyl-C₁₋₄-alkylene, especially benzyl or 2-phenylethyl.

When R³ or R⁴ is alkoxy it preferably contains up to 20, more preferably up to 10 and especially up to 4 carbon atoms; when R³ or R⁴ is halogen it is preferably fluoro or chloro.

When any one of the groups represented by R¹, R², R³, R⁴, R⁵ and R⁶ is substituted, preferred substituents are selected from alkoxy, nitro, halogen, alkoxyalkoxy, cyclohexyl, phenyl, diphenyl, hydroxy, alkylcarbonyl, alkoxycarbonyl, alkoxyalkoxycarbonyl, alkylcarbonyloxy, alkylcarbonylamino, cyano, amino, alkylamino and dialkylamino in which each alkyl is preferably C₁₋₄-alkyl and each alkoxy is preferably C₁₋₄-alkoxy.

When R¹ and R² together with the N atom to which they are attached form a heterocyclic ring this is preferably heteroalicyclic. The term heteroalicyclic means a saturated or partially unsaturated ring having at least one hetero i.e. non carbon atom in the ring, such as piperidine or morpholine. When R¹ or R² together with the N atom to which they are attached, R³ or R⁴ and the adjacent carbon atom of the benzene ring to which R³ or R⁴ is attached form a heterocyclic ring fused to the benzene ring this is preferably heteroalicyclic, an example, together with the fused benzene ring being tetrahydroquinoline.

The compounds of Formula (1) give blue shades when applied by disperse dyeing processes to synthetic fibres, such as polyesters and which exhibit good levelness.

A further preferred sub-group of polycyclic dyes of Formula (1) is that in which R⁵ and R⁶ are both -H and R¹, R², R³ and R⁴ are as hereinbefore defined.

A further preferred sub-group of polycylic dyes of Formula (1) is that in which R⁵ and R⁶ are both -H and R¹, R², R³ and R⁴ are each independently -H or optionally substituted alkyl provided that if R³ and R⁴ are both alkyl then R¹ or R² is -H.

A further preferred sub-group of polycyclic dyes of Formula (1) is that in which R⁵ and R⁶ are both -H and R¹, R², R³ and R⁴ are each independently -H or optionally substituted alkyl provided that only two of the groups represented by R¹, R², R³ and R⁴ are alkyl. The preferred sub-groups of compounds of Formula (1) also give blue shades when applied to synthetic fibres, such as polyesters by disperse dyeing processes which build up well to deep navy shades and exhibit good levelness and excellent light fastness.

Further preferred groups of polycyclic dyes of Formula (1) are those in which R¹ is -H, R², R³ and R⁴ each independently is -H or C₁₋₄-alkyl, R⁵ and R⁶ are -H; or in which R¹, R², R⁵ and R⁶ are -H, R³ and R⁴ are C₁₋₄-alkyl; or in which R¹, R², R⁴, R⁵ and R⁶ are -H and R³ is C₁₋₄-alkyl; or in which R¹, R⁴, R⁵ and R⁶ are -H and R² and R³ are C₁₋₄-alkyl or in which R¹, R², R⁵ and R⁶ are -H, R³ is -H or C₁₋₄-alkoxy and R⁴ is C₁₋₄-alkoxy.

Especially preferred polycyclic dyes of Formula (1) are those in which R¹, R², R⁵ and R⁶ are -H, R³ is methyl and R⁴ is ethyl; or in which R¹, R², R⁴, R⁵ and R⁶ are -H and R³ is ethyl; or in which R¹, R², R⁴, R⁵ and R⁶ are -H and R³ is methyl; or in which R¹, R⁴, R⁵ and R⁶ are -H, R² is ethyl and R³ is methyl; or in which R¹, R², R³, R⁵ and R⁶ are -H and R⁴ is C₁₋₄-alkoxy.

Polycyclic dyes of Formula (1) may be made by reaction of a tartronic acid with a benzofuranone in the presence of an acid such as acetic acid optionally in the presence of a strong acid such as sulphuric acid at a temperature from ambient to 100°C followed by an oxidation, with an oxidising agent such as a persulphate or hydrogen peroxide, to dehydrogenate the intermediate compound. The product may be isolated, after cooling and diluting with water, by filtration. Examples of such preparations may be found in European Patent Application No. 0363034 or US Patent No. 5,084,580. The benzofuranone referred to above may be prepared by reaction of a mandelic acid with a hydroquinone in an acid, such as 70% sulphuric acid or acetic/sulphuric acid mixtures, at elevated temperatures, the product may be isolated by filtration after diluting the reaction mixture with water. Examples of such preparations may be found in US Patent No. 4,650,882.

The invention is further illustrated by the following Examples in which all parts and percentages are by weight unless otherwise indicated.

### Example 1

A mixture of 5-hydroxy-2-oxo-3-(4-hydroxyphenyl)-2,3-dihydrobenzofuran (4.5 parts), 4-amino-3-ethyl-5-methylphenyltartronic acid (8.56 parts) and acetic acid (50 parts) was stirred under reflux for 3 hours. After cooling to ambient temperature, the mixture was diluted with water (30 parts). The product was isolated by filtration, washed with water and dried. Purification of the product was achieved, by stirring with ethanol (250 parts) for 2 hours and filtration to yield 3-(4-hydroxy-phenyl)-7-(4-amino-3-ethyl-5-methylphenyl)-2,6-dioxo-2,6-dihydrobenzo-[1:2-b, 4:5-b']difuran (1.91 parts) λ max = 580nm; Emax = 40,000 (CH₂Cl₂).

When applied to a polyester textile material from an aqueous dispersion it gave strong navy blue shades, with high light fastness and good levelling properties.

### Example 2

A mixture of 5-hydroxy-2-oxo-3-(4-hydroxyphenyl-2,3-dihydrobenzofuran (6.0 parts), 4-amino-3-ethylphenyltartronic acid (11.7 parts) and acetic acid (70 parts) was stirred under reflux for 6 hours. After cooling to ambient temperature, the mixture was diluted with water (40 parts). The product was isolated by filtration, washed with water and dried. Purification of the product was achieved, by stirring with ethanol (250 parts) for 2 hours and filtration to yield 3-(4-hydroxy phenyl)-7-(4-amino-3-ethylphenyl)-2,6-dioxo-2,6-dihydrobenzo[1:2-b, 4:5-b'] difuran (3.0 parts) λmax = 570nm; Emax = 37,500 (CH₂Cl₂).

When applied to a polyester textile material from an aqueous dispersion it gave strong navy blue shades, with high light fastness and good levelling properties.

### Example 3

A mixture of 5-hydroxy-2-oxo-3-(4-hydroxyphenyl)-2,3-dihydrobenzofuran (5.0 parts), 4-(N-ethylamino)-3-methylphenyltartronic acid (10.2 parts) and acetic acid (70 parts) was stirred under reflux for 3 hours. After cooling to ambient temperature, the mixture was diluted with water (50 parts). The product was isolated by filtration, washed with water and dried. Purification of the product was achieved, by stirring with ethanol (250 parts) for 2 hours and filtration to yield 3-(4-hydroxy-phenyl)-7-(4-(N-ethylamino)-3-methylphenyl)-2,6-dioxo-2,6-dihydrobenzo-[1:2-b, 4:5-b']difuran (3.0 parts) λmax = 620nm; Emax = 41,000 (CH₂Cl₂).

When applied to a polyester textile material from an aqueous dispersion it gave strong navy blue shades, with high light fastness and good levelling properties.

### Example 4

A mixture of 5-hydroxy-2-oxo-3-(4-hydroxyphenyl)-2,3-dihydrobenzofuran (24.2 parts), 4-amino-3-methoxyphenyltartronic acid (91.0 parts) and acetic acid (290.4 parts) was stirred for 4 hours. The reaction mixture was cooled to 80°C and water (500 parts) was added before cooling to ambient temperature. The precipitated product was collected by filtration and washed with water and petroleum spirit (bp 60-80°C) to give 30.2 parts of product. The product was stirred under reflux with ethyl cellosolve (648 parts), hydrochloric acid (130 parts, 36% w/w) and water (130 parts) for 100 minutes before diluting with water (500 parts), cooling to ambient temperature and collecting the precipitated product by filtration. The product was slurried in water (1500 parts) and the pH was adjusted to 9.0 with aqueous ammonia (35% w/w) and the mixture was stirred at ambient temperature for 16 hours. The product was isolated by filtration and dissolved in tetrahydrofuran (4000 parts) before drying over anhydrous magnesium sulphate. The tetrahydrofuran was decanted and evaporated to leave crude product (10 parts) which was further purified by elution from silica using mixtures of dichloromethane and tetrahydrofuran as eluent. 3-(4-Hydroxyphenyl)-7-(4-amino-3-methoxyphenyl)-2,6-dioxo-2,6-dihydrobenzo[1:2-b, 4:5-b']difuran (1.2 parts) λmax = 624nm, Eₘₐₓ = 39,805 (tetrahydrofuran) was obtained.

When applied to a polyester textile material from an aqueous dispersion it gave strong navy blue shades, with high light fastness and good levelling properties.

## Claims

1. A polycyclic dye of the Formula (1): 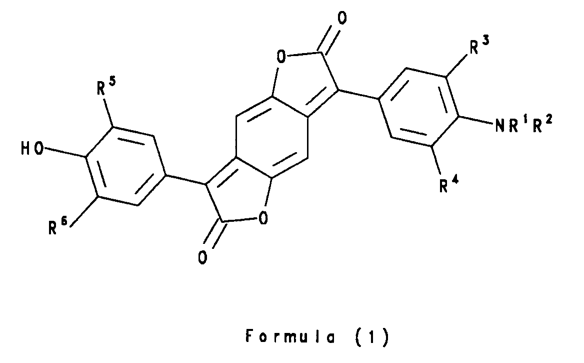 wherein:
R¹ and R² each independently is alkyl, alkenyl, cycloalkyl, aryl or aralkyl each of which may be optionally substituted or -H; or
R¹ and R² together with the N atom to which they are attached form a heterocyclic ring; or
R¹ and R² together with the N atom to which they are attached and the adjacent carbon atom of the benzene ring to which R³ or R⁴ is attached form a heterocyclic ring fused to the benzene ring;
R³ and R⁴ each independently is -H or an optionally substituted alkyl group, an optionally substituted alkoxy group or halogen provided that if both R³ and R⁴ are alkyl or alkoxy that one of R¹ and R² is H; and
R⁵ and R⁶ each independently is -H or optionally substituted alkyl.

2. A polycyclic dye according to Claim 1 wherein R¹ and R² each independently is alkyl, alkenyl, cycloalkyl, aryl or aralkyl each of which may be optionally substituted.

3. A polycyclic dye according to Claim 1 wherein R⁵ and R⁶ are both -H.

4. A polycyclic dye according to Claim 1 wherein R¹, R², R³ and R⁴ are each independently -H or optionally substituted alkyl and R⁵ and R⁶ are both -H provided that when R³ and R⁴ are both alkyl R¹ or R² is -H.

5. A polycyclic dye according to Claim 1 wherein R¹, R², R³ and R⁴ are each independently -H or optionally substituted alkyl and R⁵ and R⁶ are both -H provided that only two of the groups represented by R¹, R², R³ and R⁴ are alkyl.

6. A polycyclic dye according to Formula (1) wherein R¹, R⁵ and R⁶ are -H, R², R³ and R⁴ each independently is -H or C₁₋₄-alkyl.

7. A polycyclic dye according to Formula (1) wherein R¹, R², R⁵ and R⁶ are -H and R³ and R⁴ are C₁₋₄-alkyl.

8. A polycyclic dye according to Formula (1) wherein R¹, R², R⁴, R⁵ and R⁶ are -H and R³ is C₁₋₄-alkyl.

9. A polycyclic dye according to Formula (1) wherein R¹, R⁴, R⁵ and R⁶ are -H and R² and R³ are C₁₋₄-alkyl.

10. A polycyclic dye according to Formula (1) wherein R¹, R², R⁵ and R⁶ are -H, R³ is -H or C₁₋₄-alkoxy and R⁴ is C₁₋₄-alkoxy.
